# EUROPEAN PATENT SPECIFICATION

(11) **EP 2 301 489 B1**
(45) Date of publication and mention of the grant of the patent: **09.10.2019**
(21) Application number: 08757544.5
(22) Date of filing: 29.05.2008
(51) Int. Cl.: A61F 5/045, A61H 1/00, A61G 13/00, A61H 1/02

(54) **SPINAL THREE-DIMENSIONAL ORTHOPAEDIC EQUIPMENT**
DREIDIMENSIONALE ORTHOPÄDISCHE VORRICHTUNG FÜR DIE WIRBELSÄULE
DISPOSITIF ORTHOPÉDIQUE TRIDIMENSIONNEL POUR LA COLONNE VERTÉBRALE

(43) Date of publication of application: 30.03.2011
(73) Proprietor: ZHANG, Jilin, Jinan, Shandong 250002 (CN)
(72) Inventor: ZHANG, Jilin, Beijing 100083 (CN); ZHANG, Yi, Beijing 100083 (CN)
(74) Representative: Intès, Didier Gérard André
(86) International application number: PCT/CN2008/071133
(87) International publication number: WO 2009/143673

(56) References cited:
- WO-A1-90/03770
- CN-A- 1 149 443
- CN-C- 1 101 173
- US-A- 4 649 905
- US-A- 4 960 111
- US-A- 5 192 306
- US-A- 5 500 002
- US-A1- 2005 216 061
- US-B1- 6 328 759
- US-B1- 6 934 988
- US-B1- 6 971 997
- US-B2- 7 127 757

## Description

### TECHNICAL FIELD

The present invention relates to medical appliances and in particular to a medical appliance for remedying parenchyma trauma between spinal vertebras.

### BACKGROUND OF THE INVENTION

Chinese patent (patent number: 97182091.0) disclosed a three-dimensional spine remedying apparatus for remedying the pathological changes caused by three-dimensional displacements between spinal vertebras merged with intervertebral parenchyma trauma. The three-dimensional spine remedying apparatus comprises a frame for mounting operating mechanisms and drive devices of the orthopedic equipment; a cephalothoracic board, which is fixed on said frame, for supporting and securing the upper torso of the patient, and capable of moving horizontally along the longitudinal axle (X) of the equipment; and a device for driving said cephalothoracic board; a hip-leg board, which is fixed on said frame, for supporting and securing the lower torso of the patient, and capable of rotating around the lateral axle (Y) and the longitudinal axle (X) of said apparatus; a device for rotating said hip-leg board around the axle Y; and a device for rotating said hip-leg board around the axle X; and an electric control system for controlling the moving speed and distance of foregoing mechanisms; a hip board fixed on said hip-leg board, which is capable of rotating around the vertical axle (Z) of said equipment and a device for driving said hip board. The spinal three-dimensional orthopedic equipment of the prior art can generate good treatment effect for such diseases as lumbar disc protrusion, thoracic vertebra/lumbar vertebra joint disorder sickness and lumbar muscle strain. This patent document also mentioned that the three-dimensional spine remedying apparatus can be used to treat cervical spondylosis. However, in the process of realizing the present invention the inventor discovered that, the three-dimensional spine remedying apparatus features no obvious difference from the traditional traction method in the treatment of cervical spondylosis, and fails to stably fix the patient's head and achieve the location and three-dimensional coordinated actions of head and neck, leading to long treatment time and dissatisfactory treatment effect. For the treatment of cervical spondylosis, one of the most effective methods is to apply mechanical force to realize the movement between vertebras in the three-dimensional directions, correct the vertebrae malposition or misplacement and to release the intervertebral strained relation, so that the vertebras can return or approach their natural state. However, the spinal three-dimensional orthopedic equipment of the prior art fails to provide such movement. In the process of remedying parenchyma trauma between spinal vertebras by using said spinal three-dimensional orthopedic equipment, its drive devices can realize three-dimensional rotation actions, the traction action in longitudinal direction as well as the action of transverse shearing, but does not provide the action of up-and-down shearing that can produce obvious effect in treatment. Only by means of such action of up-and-down shearing, it is feasible to achieve the three-dimensional ridge correction in real significance.

United States Patent US 4,960,111 discloses another medical apparatus illustrating the preamble of appended claim 1.

### SUMMARY OF THE INVENTION

It is the technical object of the present invention to provide a new omnibearing spinal three-dimensional orthopedic equipment for remedying the pathological changes caused by three-dimensional displacements between spinal vertebras more effectively, so as to overcome the deficiency of the prior art.

The following technical solutions are adopted to achieve the objective of the present invention and to address its technical issues.
a spinal three-dimensional orthopedic equipment according to claim 1 includes a frame for fixing an operation means and a drive device, a front board member and its drive device fixed on the frame, a back board member and a middle board member as well as their drive devices fixed on the frame.

Wherein the spinal three-dimensional orthopedic equipment also includes a head holding means movably placed on the middle board member for holding the patient's head.

Preferably, for the spinal three-dimensional orthopedic equipment, the head holding means includes a splint connected to the middle board member movably, several sliding grooves provided on the splint, and two head holding clips mounted in the two sliding grooves respectively, which can slide along the sliding grooves.

Preferably, the spinal three-dimensional orthopedic equipment also includes a front board member fast lifting device, and a drive device for controlling the lifting device.

Preferably, the spinal three-dimensional orthopedic equipment also includes a backup board, the backup board and the front board member jointly form a drawing-pulling structure or a movable structure which can be attached and detached.

Preferably, the spinal three-dimensional orthopedic equipment also includes two grooves placed for supporting soft and elastic substance, which are placed in the front board member and on the positions corresponding to two breasts of a patient taking the prone position for treatment. Preferably, for the spinal three-dimensional orthopedic equipment, a pressure sensing device is provided on the front board member.

Preferably, for the spinal three-dimensional orthopedic equipment, the drive device for driving the lifting device of the front board member is achieved by using a motor to drive a cam.

Preferably, the spinal three-dimensional orthopedic equipment also includes a control computer connected to the pressure sensing device.

As compared with the prior art, the present invention has obvious advantages and beneficial effects. By adopting the spinal three-dimensional orthopedic equipment of the present invention, it is feasible to achieve the three-dimensional ridge correction in real significance, automatically treat the patient with parenchyma trauma between spinal vertebras or rehabilitation of human body.

Said description merely provides the overview of the technical solution of the present invention. The present invention is described herein based on preferred embodiments in combination with the attached drawings, so that the technical means of the present invention can be understood more clearly and implemented in accordance with the content of the product description, and the objectives, characterizes and advantages of the present invention are easier for understanding.

### BRIEF DESCRIPTION OF THE FIGURES

Further detailed description of the present invention is provided herein in combination with the attached figures.
FIG.1 is the structural diagram of the spinal three-dimensional orthopedic equipment of the present invention.
FIG.2 is the top view illustrating the head holding means of the spinal three-dimensional orthopedic equipment of the present invention.
FIG.3 is the schematic diagram illustrating the operating state of the head holding means of the spinal three-dimensional orthopedic equipment of the present invention.
FIG.4 is the top view illustrating the front board member of the spinal three-dimensional orthopedic equipment of the present invention.

### Detailed Description of the Preferred Embodiments

To further elaborate the technical means adopted to achieve the objective and effect of the present invention, the spinal three-dimensional orthopedic equipment and its specific structure, application method and effect of the present invention is further described in detail in combination with the attached drawings and preferred embodiments.

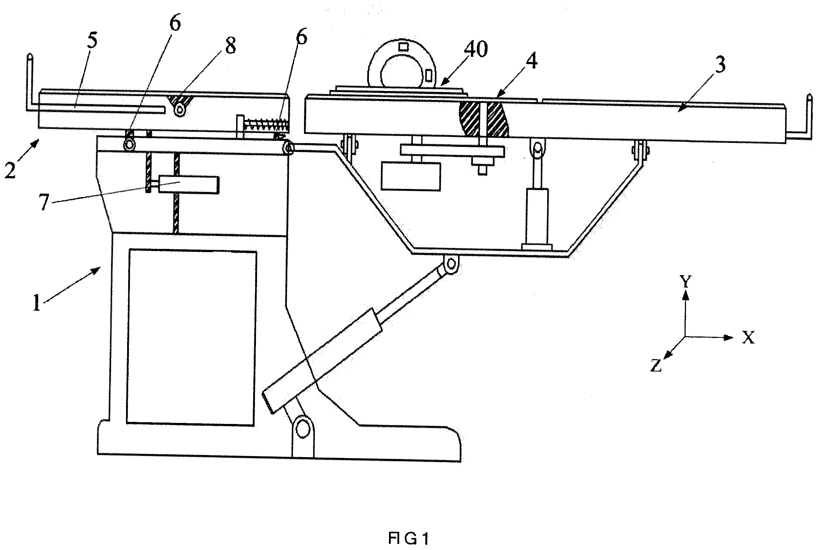

FIG.1 is the structural diagram of the spinal three-dimensional orthopedic equipment of the present invention. The spinal three-dimensional orthopedic equipment includes a frame 1 for mounting operating mechanisms and drive devices of the orthopedic equipment; a front board member 2, which is mounted on the frame 1, for supporting and securing the upper torso of the patient, and capable of rotating around the longitudinal axle (namely, X direction) of the orthopedic equipment; a drive device for driving the front board member 2 to make fast movement; a back board member 3, which is mounted on the frame 1, for supporting the lower torso of the patient in remedying the parenchyma trauma between thoracic/lumbar vertebras , and capable of rotating around the lateral axle (namely, Y direction) of the orthopedic equipment; a drive device for driving the backboard member 3 to rotate around Y direction axle; a drive device for driving the back board member 3 to rotate around X-direction axle; a middle board member 4, which is mounted on the back board member 3, capable of rotating around the axle being perpendicular to the back board surface of the orthopedic equipment(namely Z-direction axle), for implementing transverse shearing between the vertebras with pathologic changes of the patient; a drive device for driving the middle board member 4 to rotate around Z-direction axle; as well as an electric control system for controlling the movement velocities and distances of operating mechanisms (not shown in the FIGs). In the treatment of cervical spondylosis, a head holding means 40 is mounted on the middle board member 4, which is used to hold the head of the patient and thus treat the cervical spondylosis of patient.

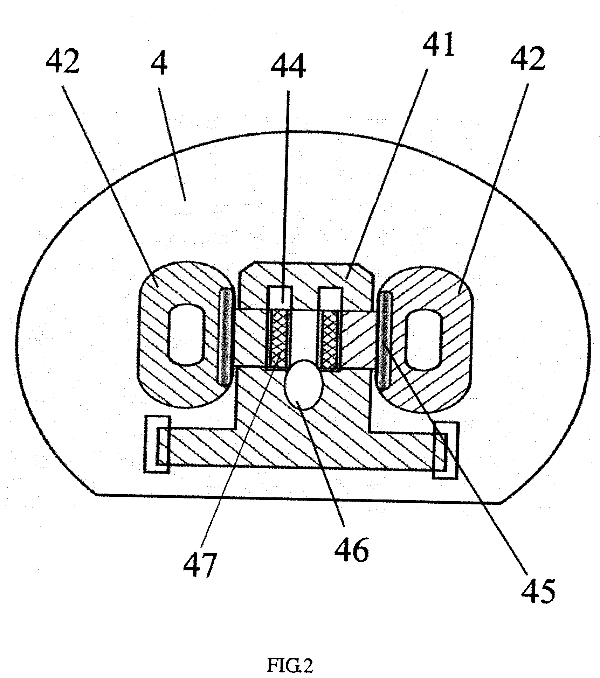

As shown in FIG.2, the head holding means 40 includes a splint 41, holding clips 42 and fastening strap 43. The splint 41 is tabular, below which a leg-shaped structure is provided, its leg is inserted and fixed to the strap hole of the middle board member 4 and can make synchronized movement along with the movement of the middle board member 4; the fastening strap 43 and its quantitative tensioning device have been described in the aforementioned patient. Two sliding grooves 44 are provided on the splint 41, and two head holding clips 42 are provided to hold the head of the patient, each holding clip has a rotating shaft 45; the rotating shaft 45 is connected to the sliding pieces 47 mounted in the sliding groove 44 and a mandible tray 46, so that the holding clips together with the mandible tray 46 can make longitudinal sliding motion in the sliding groove 44. In addition, two holding clips also make open and close movement around the rotating shaft 45. Ear holes are provided on the holding clips 42, which are used to accommodate the ears in securing the head of the patient to prevent the ears of patient from injury due to extrusion. At the same time, the ear holes can also guarantee satisfactory hearing acuity when the patient is accepting treatment. The fastening strap 43 is mounted on the middle board member 4, and is used for fastening the pelvis in remedying the disease between lumbar vertebras and for securing the head of patient placed on the splint 41 in the treatment of cervical spondylosis. The method is to use the fastening strap 43 to wrap two holding clips 42 and allow them to secure the head of patient, so that the head of patient can be stably secured on the head holding means. It is preferred that the fastening strap 43 is a device with adjustable tensioning force.

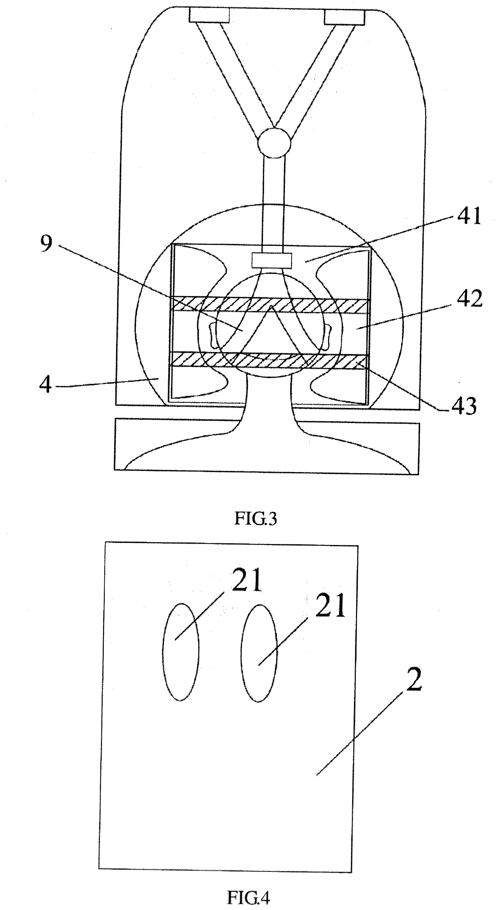

FIG.3 is the schematic diagram when the head of patient is secured on the head holding means 40 for treatment. On the head holding means 40, under the fixation effect of a longitudinal traction belt 9 and the head holding means 40, the head of patient can stably move in together with the middle board member 4 and can accept the three-dimensional coordinated actions of three quantitative boards.

For the treatment of disease between vertebras, the patient is required to take the prone position on the front board. In this case, the chest and breasts of a female patient will be subject to extrusion, which may cause adverse consequence. As shown in FIG.4, two grooves 21 are set on the front board 2 of the spinal three-dimensional orthopedic equipment, and soft elastic material is filled in the grooves 21, or, on the positions in the front board member where two breasts are located when the patient is taking the prone position for treatment, a sag lifting device and its driving mechanism are provided to accommodate the chest and breasts of the patient in the process of treatment, so as to improve the comfortableness of the spinal three-dimensional orthopedic equipment in treatment.

As shown in FIG.1, for the spinal three-dimensional orthopedic equipment of the prior art, since its front board member is mainly used for supporting the upper torso of the patient in the treatment of lumbar disease, its length is limited. In the treatment of cervical spondylosis, the front board member 2 is used for supporting the whole torso below the head of patient. Therefore, a backup board 5 is provided in the front board member of the spinal three-dimensional orthopedic equipment of the present invention, the backup board 5 and the front board member 2 jointly form a drawing-pulling structure or a movable structure which can be attached and detached. In the process of treatment, the backup board 5 is drawn out or attached to support the legs and feet of the patient. Upon completion of treatment, the backup board 5 can be pushed into the front board member 2 or detached, so that the spinal three-dimensional orthopedic equipment can be used for treating the patient with thoracic vertebra/lumbar vertebra disease.

In order to treat the disease between vertebras more effectively, it is necessary to move the vertebras with pathologic changes up and down in the process of treatment, so as to generate infinitesimal displacement of them in vertical direction. To realize said objective, a fast lifting device 6 and a drive device 7 for controlling the fast lifting device are mounted below the front board member, the fast lifting device 7 can be designed as a cam or an eccentric cam set below the front board member as well as its drive device.

Preferably, a pressure sensor 8 is provided on the front board member to measure the weight endured on the front board member, so as to acquire the body weight data of patient and provide data on more aspects for treatment. The pressure sensor is connected with computer and thus can be used as a basis for automatic setup of treatment parameters, and can also make counting for the treated patients. When the pressure sensor fails to detect pressure, it is believed that there is no patient, and then the action is trial idle operation of the spinal three-dimensional orthopedic equipment.

The technical characteristics that are not described in detail in this application can be founded in Chinese patent (number: 97182091.0), and the corresponding technical effect can be achieved. Therefore, unnecessary details of the corresponding content will be no longer provided herein.

The preferred embodiments of the present invention have been disclosed as above, but are not used to limit the present invention in any form. Those skilled in the art may make some changes or modifications to achieve equivalent embodiments by using the methods and technical content disclosed above. However, any simple modification, equivalent change and modification made to said embodiments based on the technical essence of the present invention without deviation from the technical solution of the present invention still falls within the claims of the present invention.

The invention is defined by the content of the claims.

### Industrial applicability

The spinal three-dimensional orthopedic equipment of the present invention can not only effectively remedy the disease between thoracic vertebras/ lumber vertebras, but can also effectively treat cervical spondylosis and used for health protection of spinal column. Furthermore, by means of the pressure sensing device, it can automatically judge whether there is a patient who is accepting treatment. Since a front board member fast lifting device is added, it is feasible to generate better treatment effect on the disease between vertebras.

## Claims

1. A spinal three-dimensional orthopedic equipment includes a frame (1) for fixing an operation means and a drive device, a front board member (2) and its drive device fixed on the frame (1), a back board member (3) and a middle board member (4) mounted on the back board member (3), as well as their respective drive devices fixed on the frame (1), **characterized in that** the spinal three-dimensional orthopedic equipment also includes a head holding means (40) movably placed on the middle board member (4) for holding and stably fixing the patient's head.

2. The spinal three-dimensional orthopedic equipment of claim 1, wherein the head holding means (40) includes a splint (41) connected to the middle board member (4) movably, several sliding grooves (44) provided on the splint (41), and two head holding clips (42) and a mandible tray (46) connected to the sliding pieces (47) mounted in the two sliding grooves (44) respectively, which can slide along the sliding grooves (44).

3. The spinal three-dimensional orthopedic equipment of claim 1 or 2, wherein the spinal three-dimensional orthopedic equipment also includes a front board member fast lifting device (6), and a drive device (7) for controlling the lifting device (6).

4. The spinal three-dimensional orthopedic equipment of any one of claims 1 to 3, wherein the spinal three-dimensional orthopedic equipment also includes a backup board (5), the backup board (5) and the front board member (2) jointly form a drawing-pulling structure or a movable structure which can be attached and detached.

5. The spinal three-dimensional orthopedic equipment of any one of claims 1 to 4, wherein the spinal three-dimensional orthopedic equipment also includes two grooves (21) placed on the front board member (2) and on the positions corresponding to two breasts of a patient taking the prone position for treatment.

6. The spinal three-dimensional orthopedic equipment of any one of claims 1 to 5, wherein a pressure sensing device (8) is provided on the front board member (2).

7. The spinal three-dimensional orthopedic equipment of claim 3 and any one of claims 1 to 6, wherein the drive device (7) for driving the lifting device (6) of the front board member (2) adopts cam or compression spring combination.

8. The spinal three-dimensional orthopedic equipment of claim 6 and any one of claims 1 to 7, wherein the spinal three-dimensional orthopedic equipment also includes a control computer connected with the pressure sensing device (8).

## Patentansprüche

1. Dreidimensionale orthopädische Vorrichtung für die Wirbelsäule, umfassend einen Rahmen (1) zur Befestigung eines Operationsmittels und einer Antriebseinrichtung, ein vorderes Tischelement (2) und seine Antriebseinrichtung, die an dem Rahmen (1) befestigt sind, ein hinteres Tischelement (3) und ein mittleres Tischelement (4), das an dem hinteren Tischelement (3) befestigt ist, sowie deren jeweilige an dem Rahmen (1) befestigte Antriebseinrichtungen, **dadurch gekennzeichnet, dass** die dreidimensionale orthopädische Vorrichtung für die Wirbelsäule ferner eine Kopfhalteeinrichtung (40) umfasst, die beweglich an dem mittleren Tischelement (4) zum Halten und stabilen Befestigen des Kopfes des Patienten angeordnet ist.

2. Dreidimensionale orthopädische Vorrichtung für die Wirbelsäule nach Anspruch 1, wobei die Kopfhalteeinrichtung (40) eine Schiene (41), die mit dem mittleren Tischelement (4) beweglich verbunden ist, mehrere an der Schiene (41) vorgesehene Gleitnuten (44), zwei Kopfhaltebügel (42) und eine mit in den beiden Gleitnuten (44) jeweils gelagerten Gleitstücken (47) verbundene Unterkieferschale (46), die entlang der Gleitnuten (44) gleiten kann, umfasst.

3. Dreidimensionale orthopädische Vorrichtung für die Wirbelsäule nach Anspruch 1 oder 2, wobei die dreidimensionale orthopädische Vorrichtung für die Wirbelsäule ferner eine Schnellhebevorrichtung (6) für das vordere Tischelement und eine Antriebseinrichtung (7) zum Steuern der Hebevorrichtung (6) umfasst.

4. Dreidimensionale orthopädische Vorrichtung für die Wirbelsäule nach einem der Ansprüche 1 bis 3, wobei die dreidimensionale orthopädische Vorrichtung für die Wirbelsäule ferner eine Sicherungsplatte (5) umfasst, wobei die Sicherungsplatte (5) und das vordere Tischelement (2) gemeinsam eine Zieh-Zugstruktur oder eine bewegliche Struktur bilden, die befestigt und abgenommen werden kann.

5. Dreidimensionale orthopädische Vorrichtung für die Wirbelsäule nach einem der Ansprüche 1 bis 4, wobei die dreidimensionale orthopädische Vorrichtung für die Wirbelsäule ferner zwei Nuten (21) umfasst, die auf dem vorderen Tischelement (2) und an den Positionen angeordnet sind, die den zwei Brüsten einer Patientin entsprechen, die zur Behandlung eine Bauchlage einnimmt.

6. Dreidimensionale orthopädische Vorrichtung für die Wirbelsäule nach einem der Ansprüche 1 bis 5, wobei eine Druckerfassungsvorrichtung (8) an dem vorderen Tischelement (2) vorgesehen ist.

7. Dreidimensionale orthopädische Vorrichtung für die Wirbelsäule nach Anspruch 3 und nach einem der Ansprüche 1 bis 6, wobei die Antriebsvorrichtung (7) zum Antreiben der Hebevorrichtung (6) des vorderen Tischelements (2) eine Nocken- oder Druckfederkombination annimmt.

8. Dreidimensionale orthopädische Vorrichtung für die Wirbelsäule nach Anspruch 6 und nach einem der Ansprüche 1 bis 7, wobei die dreidimensionale orthopädische Vorrichtung für die Wirbelsäule ferner einen Steuercomputer umfasst, der mit der Druckerfassungsvorrichtung (8) verbunden ist.

## Revendications

1. Dispositif orthopédique tridimensionnel pour colonne vertébrale comprenant un bâti (1) pour fixer un moyen de fonctionnement et un dispositif d'entraînement, un élément de panneau avant (2) et son dispositif d'entraînement fixé sur le bâti (1), un élément de panneau arrière (3) et un élément de panneau central (4) monté sur l'élément de panneau arrière (3), ainsi que leurs dispositifs d'entraînement respectifs fixés sur le bâti (1), **caractérisé en ce que** le dispositif orthopédique tridimensionnel pour colonne vertébrale comprend également un moyen de support de tête (40) placé, de manière mobile, sur l'élément de panneau central (4) pour maintenir et fixer de manière stable la tête du patient.

2. Dispositif orthopédique tridimensionnel pour colonne vertébrale selon la revendication 1, dans lequel le moyen de support de tête (40) comprend une attelle (41) raccordé à l'élément de panneau central (4) de manière mobile, plusieurs rainures de coulissement (44) prévues sur l'attelle (41) et deux attaches de maintien de tête (42) et un plateau pour mâchoire inférieure (46) raccordé aux pièces coulissantes (47) montées dans les deux rainures de coulissement (44) respectivement, qui peuvent coulisser le long des rainures de coulissement (44).

3. Dispositif orthopédique tridimensionnel pour colonne vertébrale selon la revendication 1 ou 2, dans lequel le dispositif orthopédique tridimensionnel pour colonne vertébrale comprend également un dispositif de levage rapide d'élément de panneau avant (6), et un dispositif d'entraînement (7) pour contrôler le dispositif de levage (6).

4. Dispositif orthopédique tridimensionnel pour colonne vertébrale selon l'une quelconque des revendications 1 à 3, dans lequel le dispositif orthopédique tridimensionnel pour colonne vertébrale comprend également un panneau de renfort (5), le panneau de renfort (5) et l'élément de panneau avant (2) forment conjointement une structure d'étirage - traction ou une structure mobile qui peut être fixée et détachée.

5. Dispositif orthopédique tridimensionnel pour colonne vertébrale selon l'une quelconque des revendications 1 à 4, dans lequel l'équipement orthopédique tridimensionnel pour colonne vertébrale comprend également deux rainures (21) placées sur l'élément de panneau avant (2) et dans les positions correspondant aux deux seins d'un patient en position couchée sur le ventre pour le traitement.

6. Dispositif orthopédique tridimensionnel pour colonne vertébrale selon l'une quelconque des revendications 1 à 5, dans lequel un dispositif de capteur de pression (8) est prévu sur l'élément de panneau avant (2).

7. Dispositif orthopédique tridimensionnel pour colonne vertébrale selon la revendication 3 et l'une quelconque des revendications 1 à 6, dans lequel le dispositif d'entraînement (7) pour entraîner le dispositif de levage (6) de l'élément de panneau avant (2) adopte une combinaison de came ou de ressort de compression.

8. Dispositif orthopédique tridimensionnel pour colonne vertébrale selon la revendication 6 et l'une quelconque des revendications 1 à 7, dans lequel le dispositif orthopédique tridimensionnel pour colonne vertébrale comprend également un ordinateur de contrôle raccordé avec le dispositif de détection de pression (8).
